# EUROPÄISCHE PATENTANMELDUNG

(11) **EP 2 916 120 A1**
(43) Veröffentlichungstag der Anmeldung: **09.09.2015**
(21) Anmeldenummer: 14157563.9
(22) Anmeldetag: 04.03.2014
(51) Int. Cl.: G01L 13/00, A61M 16/08, A61M 39/10, F16L 17/02, F16L 21/06, G01L 19/00, G01L 19/14, G01F 1/34, G01F 1/46

(54) **Sensorblock, Rohr sowie Herstellungsverfahren**

(71) Anmelder: seleon GmbH, 74076 Heilbronn (DE)
(72) Erfinder:
(74) Vertreter: Hellmich, Wolfgang

(57) **Zusammenfassung**

Die Erfindung betrifft einen Sensorblock (1) zum Messen von Fluidfluss oder Druck in einem Rohr (22). Der Sensorblock (1) umfasst einen Sensor (4, 24) und ein Gehäuse. Das Gehäuse weist einen elastischen Klammerteil (11, 12) auf, der so geformt ist, das er auf das Rohr in radialer Richtung aufgesteckt werden kann und aufgesteckt einen Teil des Rohres umfasst. Darüber hinaus betrifft die Erfindung ein Rohr (72), das mittels MID-Technologie hergestellt ist. Das Rohr (72) umfasst Leiterbahnen (77) und einen Sensor (24, 84), der fest mit dem Rohr (72) verbunden ist. Darüber hinaus betrifft die Erfindung ein Rohr (2; 22) mit einer Öffnung (49, 36, 46, 56, 66) in der Rohrwand, die mit einem Sterilfilter (28, 38, 48) verschlossen ist. Das Rohr (2; 22) weist ferner zwei längliche Nuten (15) zur lösbaren Befestigung eines Sensorblocks (1) auf. Die Erfindung betrifft ferner ein Rohr (72), das ein doppeltes Pitotrohr (6) aufweist. Die Erfindung betrifft ferner ein Rohr (72) mit einer Blende (86) aus porösem Material. Vor und nach der Blende (86) ist je eine Öffnung (79, 89) in der Rohrwand angebracht. Beide Öffnungen (79, 89) sind durch Sterilfilter verschlossen. Ferner betrifft die Erfindung ein Herstellungverfahren, bei dem sowohl ein erfindungsgemäßer Sensorblock (1) als auch ein erfindungsgemäßes Rohr (2; 22; 72) mit dem gleichen Maskensatz, einem gleichen Spritzgusswerkzeug und/oder einem gleichen Steuerprogrammteil hergestellt werden.

## Beschreibung

Das Gebiet der Erfindung sind Beatmungsgeräte, insbesondere Sensorbauelemente hierfür.

Die Erfindung betrifft Sensorblöcke gemäß dem Oberbegriff des Patentanspruchs 1, ein Rohr mit einem Sensor, Rohre gemäß den Oberbegriffen der Patentansprüche 12 bis 14 sowie ein Herstellungsverfahren.

Laut Wikipedia ist ein Mikrosystem ein miniaturisiertes Gerät, eine Baugruppe oder ein Bauteil, dessen Komponenten kleinste Abmessungen im Mikrometerbereich haben und als System zusammenwirken. Üblicherweise besteht ein Mikrosystem aus einem oder mehreren Sensoren, Aktoren und einer Steuerungselektronik auf einem Substrat oder Chip. Die Mikrosystemtechnik ist die Lehre von der Entwicklung der Mikrosysteme und von den Techniken zu deren Realisierung.

In der englischen Sprache sind die Abkürzungen MEMS und MOEMS für microelectro-mechanical systems bzw. micro-optoelectro-mechanical systems üblich. In asiatischen, vorrangig japanischen, Veröffentlichungen findet sich hingegen auch die erweiterte Bezeichnung micromachines. Der Einsatz von Mikrosystemen ist überall dort denkbar und sinnvoll, wo Sensoren, Aktoren und Elektronik zusammenarbeiten. Medizinprodukte sowie Produkte aus den Bereichen Sicherheitstechnik, Sport, Biowissenschaften und Logistik können mit Hilfe von Mikrosystemen vielseitiger, einfacher, intelligenter, kleiner und leistungsfähiger werden.

Die Herstellung sowohl von Druck- als auch Flusssensoren mittels Mikrosystemtechnik ist allgemein bekannt. Beispielhaft seien die Differenzdrucksensoren SDP1108 und SDP2108 der SENSIRON AG genannt. Da diese Sensoren ein thermisches Sensorelement aufweisen, handelt es sich genau genommen um Flusssensoren, die den geringen Gasfluss durch eine Kapillare messen. Im Rahmen dieser Anmeldung sollen als Differenzdrucksensoren entsprechend dem Datenblatt der SDP1108 und SDP2108 auch Flusssensoren, insbesondere thermische Massenflusssensoren, verstanden werden, die den Druckabfall an einer Kapillare messen.

Als Molded Interconnect Devices (englisch für: spritzgegossene Schaltungsträger), kurz MID, werden elektronische Bauteile bezeichnet, bei denen metallische Leiterbahnen auf spritzgegossene Kunststoffträger aufgetragen werden. Wesentliche Einsatzgebiete für die MID-Technik sind der Automobilbau, die Industrieautomatisierung, die Medizintechnik, die Hausgeräteindustrie, die Telekommunikationstechnik, die Mess- und Analysetechnik sowie die Luft- und Raumfahrt.

Die verbesserte Gestaltungsfreiheit und die Integration von elektrischen und mechanischen Funktionen in ein Spritzgussteil kann zu einer Miniaturisierung der Baugruppe führen. Rationalisierungspotential liegen in der Reduzierung der Teileanzahl durch Materialeinsparung und der Verkürzung der Prozessketten. Des Weiteren kann durch die Reduzierung der Montageschritte die Zuverlässigkeit erhöht werden.

Durch den Einsatz der MID-Technik kann der konventionelle Materialmix einer Kombination aus Leiterplatte und Mechanikkomponente, der meist aus vielen Werkstoffen besteht, durch ein metallisiertes Kunststoffteil (MID) ersetzt werden. MIDs werden aus rezyklierbaren Thermoplasten hergestellt und sind unkritischer bei der Entsorgung als konventionelle Leiterplatten. Das Basismaterial für eine Leiterplatte ist dagegen im Allgemeinen ein schwer entsorgbarer und nicht rezyklierbarer Duroplast.

Bekannt sind ferner Geräte zur Durchführung der CPAP (continuous positive airway pressure)-Therapie, vgl. WO 2004/045693 A2 mit weiteren Nachweisen. Ein CPAP-Gerät appliziert mittels eines Kompressors, vorzugsweise über einen Luftbefeuchter, über einen Schlauch und eine Nasenmaske einen positiven Überdruck bis zu etwa 30 mbar in den Atemwegen des Patienten. Dieser Überdruck soll gewährleisten, dass die oberen Atemwege im Schlaf während der gesamten Nacht vollständig geöffnet bleiben und somit keine obstruktiven Atmungsstörungen (Apnoen) auftreten (DE 198 49 571 A1).

Ferner sind Sterilfilter kommerziell erhältlich. Sie bestehen beispielsweise aus einem porösem Kunststoffkörper aus Polypropylen oder Teflon (PTFE, Polytetrafluorethylen), um Kleinstlebewesen abzufangen. Typische Bakterien können von 0,2 bis zu 4 µm groß sein. Viren sind kleiner als 0,3 µm und Bakteriophagen können sogar bis zu 0,01 µm klein sein.

Die US 7,814,907 B2 beschreibt ein System zum Applizieren von Gasen für Beatmungsgeräte. Ein Gebläse drückt Luft durch eine Befeuchtungskammer und eine Beatmungsleitung zu einem Patienten. Bei etwa einem Viertel der Länge der Beatmungsleitung von der Befeuchtungskammer aus ist die Beatmungsleitung durch eine beispielsweise T-förmige Röhre unterbrochen, die einen Anschluss für einen Sensorgehäuse aufweist. Der Sensor im Sensorgehäuse kann Luftfeuchte, Temperatur und / oder Fluss messen. Zwischen dem Inneren der Beatmungsleitung, insbesondere dem Inneren der Röhre, und dem Sensor ist ein Filter, beispielsweise ein SYMPATEX® - Film vorgesehen. Das Filter soll Staub, Dampf, Bakterien und Viren vom Sensor fernhalten. Das Filter kann an der Röhre festgeklebt werden. Die Verbindung zwischen Röhre und Sensorgehäuse kann durch ein Gewinde, eine Schnappbefestigung, eine Bajonett-Befestigung oder eine Silikondichtung hergestellt werden. Das Sensorgehäuse selbst kann sich in der Röhre befinden und selbst aus einem Rohr bestehen, dessen Enden durch Filter verschlossen sind.

Es ist Aufgabe der Erfindung einen verbesserten Sensorblock, ein verbessertes Rohr sowie ein verbessertes Herstellungsverfahren anzugeben.

Diese Aufgabe wird durch die Lehre der unabhängigen Ansprüche gelöst.

Bevorzugte Ausführungsformen der Erfindung sind Gegenstand der Unteransprüche.

Vorteilhaft an einem Sensorblock mit einem Gehäuse mit einem elastischen Klammerteil, der so geformt ist, dass er auf ein Rohr in radialer Richtung aufgesteckt werden kann und aufgesteckt einen Teil des Rohres umfasst, ist, dass der Sensorblock leicht auf das Rohr montiert werden kann. Dies ist bei der Montage z. B. von CPAP-Geräten vorteilhaft. Ferner ermöglicht es den Einsatz von Rohren als Einwegartikel bei gleichzeitiger Wiederverwendung des relativ teuren Sensorblocks. Um eine Kontamination des Sensors zu vermeiden, werden die Öffnungen des Rohres sinnvollerweise mit Sterilfiltern verschlossen.

Längliche Vorsprünge an den Klammerteilen, die sich parallel zur Achse des Rohres erstrecken, und/oder entsprechende Nuten im Rohr ermöglichen eine reproduzierbare Positionierung des Sensorblocks auf dem Rohr.

Der Einsatz eines Differenzdrucksensors ermöglicht in vorteilhafter Weise den Einsatz von Staurohren oder Blenden zur vorzeichenrichtigen Messung des Gasflusses im Rohr.

Ein doppeltes Pitotrohr ermöglicht im Gegensatz zu einem einfachen Pitotrohr oder einem Prandtl'schem Staurohr ebenfalls die vorzeichenrichtige Messung des Gasflusses unabhängig von der Flussrichtung im Rohr.

Ein Anschluss für einen Schlauch ermöglicht in vorteilhafter Weise das Zuführen von weiteren Gasen, insbesondere Sauerstoff oder das Ziehen von Proben aus der Atemluft.

Ein Differenzdrucksensor, der den Druckunterschied zwischen dem Anschluss des Schlauches und dem Inneren des Rohres oder der Umgebung des Rohres misst, ermöglicht in vorteilhafter Weise eine Flussmessung des über den Anschluss Zugeführten oder Entnommenen Gases. Diese Flussmessung wird genauer, wenn der Anschluss über eine Kapillaröffnung mit dem Inneren des Rohres verbunden ist.

Die Flussmessung im Rohr kann genauer sein, wenn der Differenzdruck an einer Blende (86) aus porösem Material gemessen wird, als wenn ein doppeltes Pitotrohr eingesetzt wird. Der Grund hierfür ist das flussabhängige Strömungsprofil im Rohr.

Ein Sensorblock sowie ein Rohr können in vorteilhafter Weise so designed werden, dass beide mit einem einzigen Maskensatz in MID-Technologie, zumindest teilweise gleichen Spritzgusswerkzeugen und zumindest teilweise gleichen Steuerprogrammteilen hergestellt werden können.

Im Folgenden werden bevorzugte Ausführungsformen der Erfindung unter Bezugnahme auf die beiliegenden Zeichnungen näher erläutert. Dabei zeigen:
Fig. 1 ein erfindungsgemäßes Strömungsrohr eines CPAP-Geräts;
Fig. 2 eine Explosionsdarstellung des in Figur 1 dargestellten Strömungsrohrs;
Fig. 3 eine Explosionsdarstellung eines in den Figuren 1 und 2 dargestellten, erfindungsgemäßen Sensorblocks;
Fig. 4 einen Querschnitt durch eine zweite Ausführungsform eines erfindungsgemäßen Sensorblocks und eines erfindungsgemäßen Strömungsrohrs;
Fig. 5 zeigt einen Längsschnitt durch eine dritte Ausführungsform eines erfindungsgemäßen Sensorblocks und eines erfindungsgemäßen Strömungsrohrs;
Fig. 6 zeigt einen Querschnitt durch die dritte Ausführungsform eines erfindungsgemäßen Sensorblocks und eines erfindungsgemäßen Strömungsrohrs entlang der Linie A-A in Fig. 5;
Fig. 7 zeigt einen Querschnitt durch eine vierte Ausführungsform eines erfindungsgemäßen Strömungsrohrs;
Fig. 8 zeigt einen Längsschnitt durch eine fünfte Ausführungsform eines erfindungsgemäßen Strömungsrohrs;
Fig. 9 zeigt einen Querschnitt durch eine sechste Ausführungsform eines erfindungsgemäßen Strömungsrohrs; und
Fig. 10 zeigt einen Querschnitt durch eine siebte Ausführungsform eines erfindungsgemäßen Strömungsrohrs.

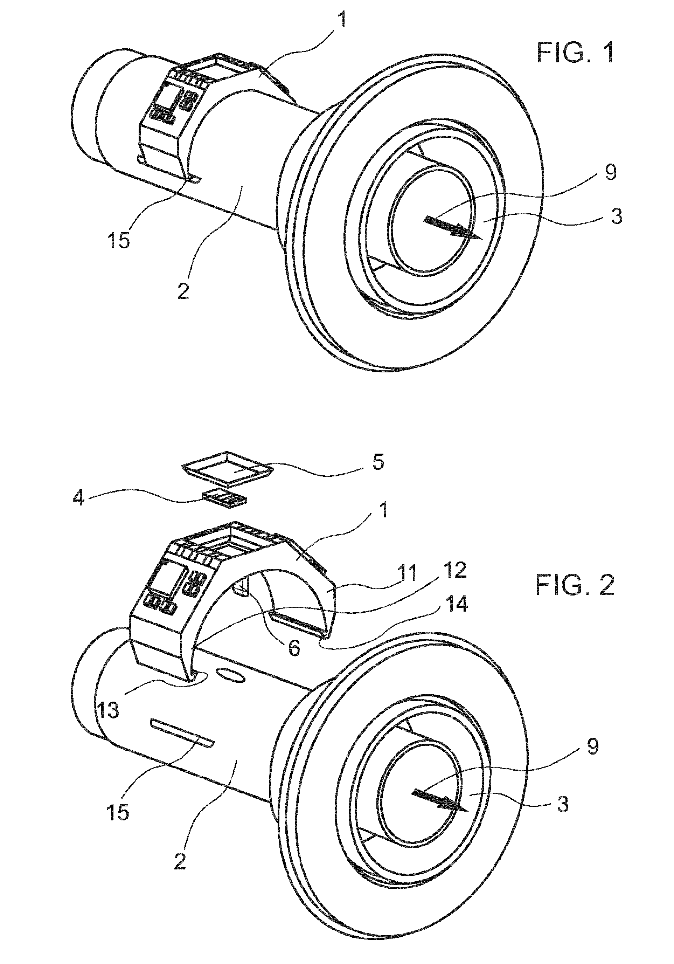

Fig. 1 zeigt ein erfindungsgemäßes Strömungsrohr 2 für ein CPAP-Gerät. Das vordere, verbreitete Ende des Strömungsrohrs 2 wird in einer Wand des CPAP-Geräts befestigt. Dort befindet sich auch der Beatmungsschlauchanschluss 3, der nach dem Zusammenbau des CPAP-Geräts von außen zugänglich bleibt. Oben auf dem Strömungsrohr 2 sitzt ein Sensorblock 1. Der Sensorblock 1 kann Sensoren zur Fluss- und / oder Druckmessung im Strömungsrohr 2 umfassen.

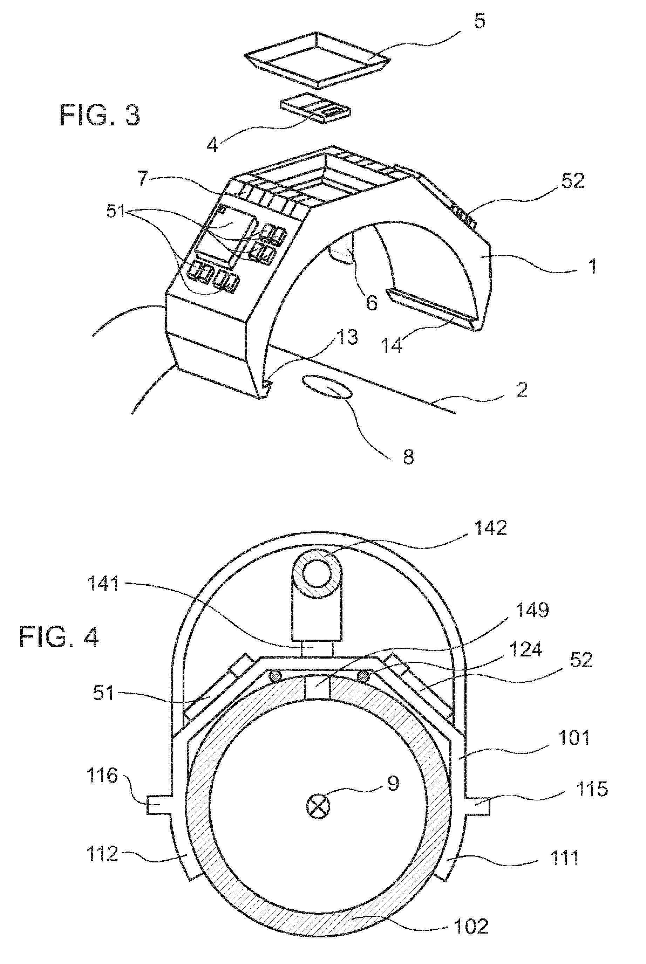

Die Fig. 2 zeigt den abgenommenen Sensorblock 1. Fig. 3 zeigt den Ausschnitt aus Fig. 2 mit dem Sensorblock 1. Der Sensorblock 1 ist C-förmig. Die beiden Enden werden als Klammerteile 11 und 12 bezeichnet. Der Sensorblock 1 mit seinen beiden Klammerteilen 11 und 12 umfasst etwas mehr als die Hälfte oder, mit anderen Worten, etwas mehr als 180° des Strömungsrohrs 2. Für einen zuverlässigeren Halt weist jedes Klammerteil 11, 12 an seinem Ende einen längliche Vorsprung 13 bzw. 14 auf. Jeder längliche Vorsprung 13 bzw. 14 greift in eine Nut 15 am Strömungsrohr 2 ein. In den Figuren 1 und 2 ist nur die Nut 15 auf der dem Betrachter zugewandten Seite des Strömungsrohrs 2 eingezeichnet.

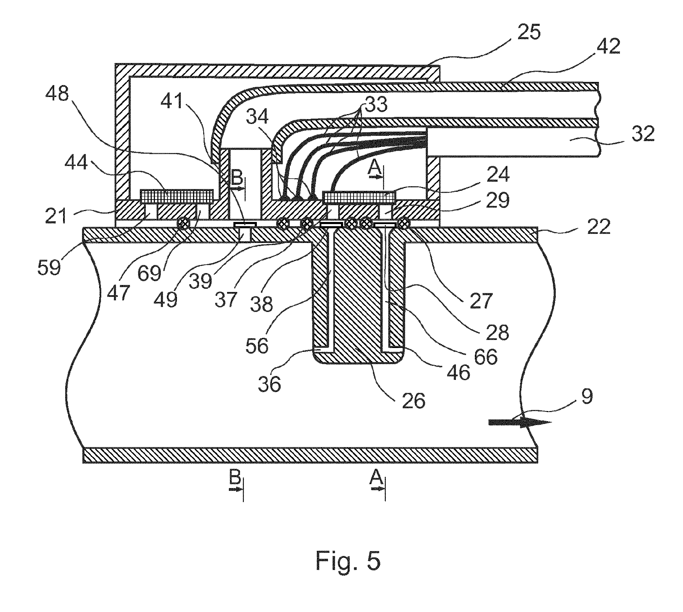

Unten ragt aus dem Sensorblock ein Pitotrohr 6 heraus. Wenn der Sensorblock 1, wie in Fig. 1 dargestellt, auf das Strömungsrohr 2 aufgesteckt ist, ragt das Pitotrohr 6 durch die Öffnung 8 in das Innere des Strömungsrohrs 2 hinein. Der Aufbau des Pitotrohrs 6 ähnelt dem Aufbau des in Fig. 5 dargestellten Pitotrohrs 26 mit zwei Öffnungen, wobei eine Öffnung in Flussrichtung 9 und eine Öffnung entgegen der Flussrichtung 9 gerichtet ist. Im Fall des Strömungsrohrs 2 ist die Flussrichtung 9 auf den Beatmungsschlauchanschluss 3 hin gerichtet.

Bei so genannten Einschlauchbeatmungsgeräten, wie sie beispielsweise CPAP-Geräte darstellen, ist in oder nahe bei der Beatmungsmaske ein definiertes Leck vorgesehen, über das das ausgeatmete Kohlendioxyd ausgewaschen wird. Über einen Atemzug gemittelt ergibt sich also ein Nettofluss vom Beatmungsgerät zur Beatmungsmaske hin in Richtung der Flussrichtung 9. Trotzdem kann beim Ausatmen Luft zum CPAP-Gerät entgegen der Flussrichtung 9 zurückgedrückt werden.

Das Strömungsrohr 2 und/oder der Sensorblock 1 sind aus einem elastischen Material hergestellt, so dass der Sensorblock 1 auf das Strömungsrohr 2 mehrmals aufgesteckt und wieder abgenommen werden kann. Der Sensorblock 1 ist in MID-Technologie ausgeführt. Auf einem Kunststoffkörper sind Leiterbahnen 7 beispielsweise aus Kupfer sowie elektronische Bauteile an den Leiterbahnen befestigt. Beispielhaft sind in Fig. 3 die Bauteile für einen Vorverstärker 51 sowie ein weiteres elektrisches Bauteil 52 dargestellt. Die Befestigung erfolgt beispielsweise durch Löten, insbesondere Schwalllöten (englisch: flow soldering) oder Kleben beispielsweise mit Silberleitkleber. Diese Art der Befestigung ist nicht nur mechanischer Natur, sondern stellt auch einen elektrischen Kontakt zwischen den elektronischen Bauteilen und den Leiterbahnen 7 sicher.

Insbesondere ist auf dem Sensorblock 1 ein Differenzdrucksensor 4 sowie eine Abdeckung 5 angebracht. Wie oben erwähnt, sollen im Rahmen dieses Dokuments als Differenzdrucksensoren auch Flusssensoren, insbesondere thermische Massenflusssensoren, verstanden werden, die den Druckabfall an einer Kapillare wie die Sensoren SDP1108 und SDP2108 messen.

Um die Leiterbahnen 7 und die elektronischen Bauteile im zentralen Teil des Sensorblocks 1 mechanisch möglichst wenig zu belasten, ist der zentrale Teil des Sensorblocks 1 steifer als die Klammerteile 11 und 12. Dies kann dadurch erreicht werden, dass der zentrale Teil dicker und / oder aus einem steiferen Material als die Klammerteile 11, 12 besteht. Falls die Klammerteile 11, 12 mehr als 180° des Strömungsrohres 2 umfassen, sind die Nuten 15 und die Vorsprünge 13, 14 nicht erforderlich. Die richtige Positionierung des Sensorblocks 1 gegenüber dem Strömungsrohr 2 kann in diesem Fall auch durch das Pitotrohr 6 und die Öffnung 8 sichergestellt werden. Wenn die Vorsprünge 14 und 15 sowie die Nuten 15 vorhanden sind, kann der Sensorblock auch weniger als 180° des Strömungsrohres 2 umfassen. Wenn die Vorsprünge 13 und 14 einen hakenförmigen Querschnitt aufweisen, und die Nuten 15 entsprechend geformt sind, ist es ausreichend, wenn der Sensorblock 1 nur einen kleinen Winkelbereich, also 60°, 30° oder weniger des Strömungsohres 2 umfasst.

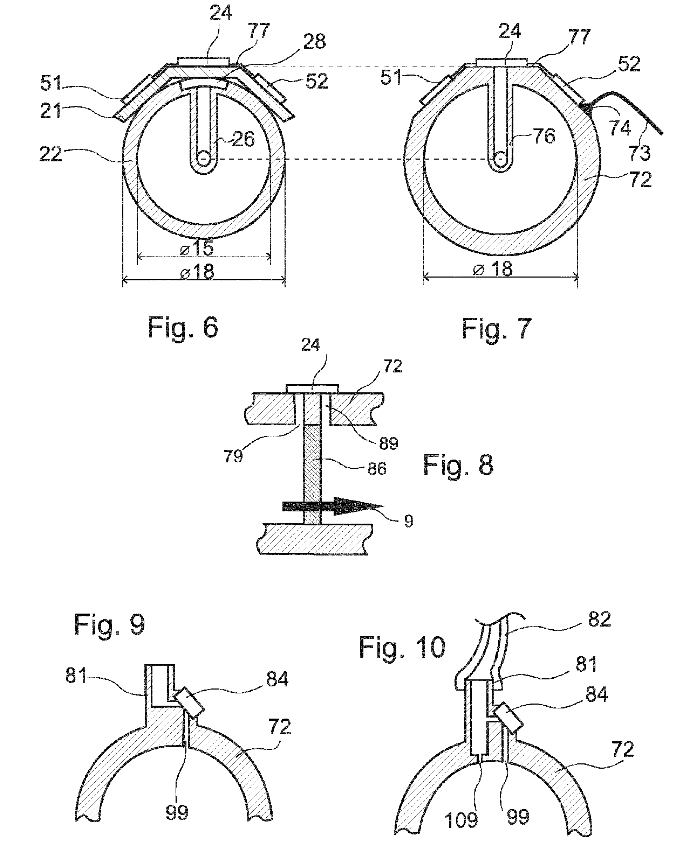

Fig. 4 zeigt einen Querschnitt durch eine zweite Ausführungsform eines erfindungsgemäßen Sensorblocks 101 und erfindungsgemäßen Strömungsrohrs 102 mit Vorverstärker 51 und elektrischem Bauteil 52. Die Flussrichtung 9 verläuft in die Zeichnungsebene hinein. Der Sensorblock 101 weist außen Rippen 115 und 116 auf, die das Abnehmen und Aufstecken des Sensorblocks 101 auf das Strömungsrohr 102 erleichtern. Bei dieser Ausführungsform weisen die Klammerteile 111 und 112 keine Vorsprünge und das Strömungsrohr 102 auch keine Nuten auf. Der Sensorblock 101 umfasst etwa 240° des Strömungsrohrs 102. Der Sensorblock 101 umfasst einen Schlauchanschluss 141 für einen Schlauch 142. Mittels eines Flusssensors wird der Fluss im Schlauch 142 gemessen. Wie die Flussmessung erfolgt, wird anhand der in den Figuren 9 und 10 dargestellten Schnitte erläutert. Der Schlauch 142 kann zum Zuführen von Sauerstoff oder anderen Gasen oder Gasgemischen für diagnostische oder therapeutische Zwecke verwendet werden. Der Schlauch 142 kann ferner zum Ziehen von Proben insbesondere aus ausgeatmetem Gas verwendet werden. Im Strömungsrohr 102 ist unterhalb des Schlauchanschlusses 141 eine Öffnung 149 angebracht, die pneumatisch mit dem Schlauchanschluss 141 verbunden ist. Dichtring 124 verhindert eine pneumatische Verbindung zur Umgebung. Zur Illustration sind in Fig. 4 wieder die Bauteile für einen Vorverstärker 51 sowie ein weiteres elektronisches Bauteil 52 dargestellt.

Der in Fig. 4 dargestellte Sensorblock 101 kann durch einen zweiten Differenzdrucksensor 4 und einen Pitotrohr 6 erweitert werden, ähnlich wie das in Fig. 5 dargestellt wird. Umgekehrt kann auch der Sensorblock 1 durch einen zweiten Differenzdrucksensor und einen Schlauchanschluss 141 ergänzt werden, ähnlich wie das in Fig. 5 dargestellt ist.

Fig. 5 zeigt einen Längsschnitt durch eine dritte Ausführungsform eines erfindungsgemäßen Sensorblocks mit einer MID-Komponente 21 und eine Abdeckung 25 sowie eines erfindungsgemäßen Strömungsrohrs 22. Fig. 5 stellt im Wesentlichen eine Kombination der in den Figuren 1-3 sowie in der Fig. 4 dargestellten Sensorblöcke 1 bzw. 101 dar. Im Unterschied zu einer solchen Kombination ist der Gasraum im Strömungsrohr 22 durch die Sterilfilter 28, 38 und 48 vom Gasraum im Sensorblock 1 getrennt.

Wenn das in Fig. 5 dargestellte Strömungsrohr 22 nahe einer Beatmungsmaske eingesetzt wird, fließt durch das Strömungsrohr 22 sowohl eingeatmete als auch ausgeatmete Luft. Die Flussrichtung 9 ist also parallel zur Achse des Strömungsrohrs 22 beliebig wählbar und unabhängig von der Wahl kommen positive und negative Gasflüsse, also Gasflüsse in Flussrichtung 9 bzw. entgegen der Flussrichtung 9 vor. Betragsmäßig sind die positiven und negativen Gasflüsse in etwa gleich groß.

Um Gasflüsse in und entgegen der Flussrichtung 9 messen zu können, ist das doppelte Pitotrohr 26 symmetrisch zu einer Ebene senkrecht der Flussrichtung 9. Insbesondere weist eine Öffnung 46 in Flussrichtung und eine Öffnung 36 entgegen der Flussrichtung. Über einen Kanal 56 und eine Öffnung 39 wird der Druck bei der Öffnung 36 einem Eingang des Differenzdrucksensors 24 zugeführt. Der Kanal 56 ist über ein Sterilfilter 38 pneumatisch mit der Öffnung 39 verbunden, wobei der Dichtring 37 die Abdichtung gegenüber der Umgebung bewirkt. In ähnlicher Weise wird der Druck in der Öffnung 46 über den Kanal 66 durch die Öffnung 29 dem anderen Eingang des Differenzdrucksensors 24 zugeführt. Da ein Sterilfilter auch für kleine Luftmoleküle wie N₂, O₂ und H₂O einen gewissen Flusswiderstand darstellt, weiten sich die Kanäle 56 und 66 unmittelbar unterhalb der Sterilfilter 28 bzw. 38, deren Fläche möglichst groß gestaltet wird. Hierdurch wird der Flusswiderstand durch die Sterilfilter gering gehalten. Der Kanal 66 ist über ein Sterilfilter 28 pneumatisch mit der Öffnung 29 verbunden und durch einen Dichtring 27 gegen die Umgebung abgedichtet. Fachleuten ist bekannt, dass basierend auf der Bernoulligleichung aus dem vom Differenzdrucksensor 24 gelieferten Signal die Strömungsgeschwindigkeit berechnet werden kann. Dabei wird die Bernoulligleichung parametrisiert, also auf den konkreten Messfall angepasst. Das Vorzeichen des Gasflusses ergibt sich aus dem Vorzeichen des vom Differenzdrucksensor 24 gelieferten Signals.

Die MID-Komponente 21 weist ferner einen Schlauchanschluss 41 für einen Schlauch 42 auf. Über ein weiteres Sterilfilter 48 und eine Kapillaröffnung 49 kann so beispielsweise Sauerstoff vom Schlauch 42 in das Strömungsrohr 22 gedrückt werden. Ein weiterer Differenzdrucksensor 44 ist vorgesehen, um die Druckdifferenz an der Kapillaröffnung 49 zu messen. Der erste Druckanschluss des Differenzdrucksensors ist pneumatisch über die Öffnung 69 mit dem Inneren des Schlauchanschlusses 41 verbunden und durch den Dichtring 47 von der Umgebung abgedichtet. Da der zweite Druckanschluss des Differenzdrucksensors 44 über die Öffnung 59 mit der Umgebung und nicht mit dem Inneren des Strömungsrohrs 22 verbunden ist, misst der Differenzdrucksensor 44 nicht exakt den Differenzdruck an der Kapillaröffnung 49. Normalerweise weicht allerdings der Druck im Strömungsrohr 22 nur um einige 10 mbar vom Umgebungsdruck ab. Wenn die vom Differenzdrucksensor 44 gemessene Druckdifferenz groß gegenüber einigen 10 mbar ist, kann die Druckdifferenz zwischen dem Umgebungsdruck und dem Druck im Strömungsrohr 22 vernachlässigt werden. Meist ist allerdings der Differenzdruck zwischen dem Inneren des Strömungsrohrs 22 und Umgebung bekannt, so dass der vom Differenzdrucksensor 44 gemessene Druck um diese Druckdifferenz korrigiert werden kann.

Die Kapillaröffnung 49 kann ähnlich den Kanälen 56 und 66 unterhalb des Sterilfilters 48 aufgeweitet sein. Dies kann insbesondere beim Ziehen von Gasproben über den Schlauch 42 von Vorteil sein. Wenn Sauerstoff über den Schlauch 42 zugeführt wird, steht der Sauerstoff in der Regel unter genügend hohem Druck, so dass trotz Differenzdruck an der Kapillaröffnung 49 und dem Sterilfilter 48 der Sauerstofffluss ausreichend ist. Unter dieser Voraussetzung ist ein hoher Differenzdruck eher von Vorteil, weil ein hoher Differenzdruck genauer vom Differenzdrucksensor 44 gemessen werden kann. Deshalb ist in Fig. 5 auch keine Aufweitung der Kapillaröffnung 49 unterhalb des Sterilfilters 48 eingezeichnet.

In einer anderen Ausführungsform kann auch eine weitere Öffnung im Strömungsrohr 22 unter der Öffnung 59 vorgesehen sein. Beide Öffnungen sind bei dieser Ausführungsform über einen Dichtring pneumatisch verbunden.

Anstelle der Dichtringe 27, 37 und 47 kann auch eine einzige Dichtung beispielsweise bestehend aus weichem Material verwendet werden, die für die Anwendung in Fig. 5 drei durch Dichtungsmaterial getrennte Öffnungen aufweisen müsste.

Die MID-Komponente 21 ist in MID-Technologie hergestellt. Beispielhaft sind deshalb die Lötstellen 34 und die Leiter 33 der elektrischen Leitung 32 eingezeichnet, über die die Differenzdrucksensoren 24 und 44 mit elektrischer Energie versorgt und ausgelesen werden. Eine Abdeckung 25 schützt die Bauteile auf der MID-Komponente 21.

Fig. 6 zeigt einen Querschnitt entlang der Linie A-A in Fig. 5 mit der MID-Komponente 21, dem Strömungsrohr 22, dem doppelten Pitotrohr 26, dem Sterilfilter 28, dem Differenzdrucksensor 24, Vorverstärker 51 sowie einem elektronischem Bauteil 52.

Fig. 7 zeigt ein Strömungsrohr 72, das in MID-Technologie hergestellt ist und das doppelte Pitotrohr 76 umfasst. Die in den Figuren 5 und 6 dargestellte MID-Komponente 21 ist bei der in Fig. 7 dargestellten Ausführungsform Teil des Strömungsrohrs 72. Oben auf dem Strömungsrohr 72 sind wieder Vorverstärker 51, Differenzdrucksensor 24 und ein elektronisches Bauteil 52, Leiterbahnen 77, Lötstellen 74 sowie ein elektrischer Anschluss 73 dargestellt. Der Vorteil an der in Fig. 7 dargestellten Ausführungsform besteht darin, dass die Dimensionen des Strömungsrohrs 72 zumindest im oberen Bereich gleich der MID-Komponente 21 sind, so dass der gleiche Maskensatz, ein oder mehrere gleiche Spritzgusswerkzeuge und/oder das in einem oder mehreren Teilen gleiche Steuerprogramm für die Herstellung der MID-Komponente 21 wie für die Herstellung des Strömungsrohrs 72 verwendet werden kann. Maskensatz und Spritzgusswerkzeuge können unter dem Oberbegriff Werkzeugsatz zusammengefasst werden.

Fig. 8 zeigt einen Längsschnitt durch eine fünfte Ausführungsform eines erfindungsgemäßen Strömungsrohrs 72. Bei dieser Ausführungsform wird in bekannter Weise durch einen Gasfluss in einem Strömungsrohr 72 ein Druckunterschied an einer porösen Blende 86 erzeugt. Die Flussrichtung 9 ist von links nach rechts festgelegt. Der Druckunterschied wird über zwei Öffnungen 79 und 89 den beiden Anschlüssen eines Differenzdrucksensors 24 zugeführt. In dieser Ausführungsform ist das Strömungsrohr 72 in MID-Technologie ausgeführt. Fachleuten ist jedoch klar, dass das doppelte Pitotrohr 26 in der in Fig. 5 dargestellten Ausführungsform auch durch eine poröse Blende 86 ersetzt werden kann.

In den Figuren 9 und 10 sind alternative Ausführungsformen für den linken Teil des in Fig. 5 dargestellten Strömungsrohrs 22 entlang der Linie B-B dargestellt. In beiden Fällen ist das Strömungsrohr 72 wie in den Figuren 7 und 8 in MID-Technologie ausgeführt. In Fig. 9 ist kein Schlauch auf den Schlauchanschluss 81 aufgesteckt. Ein Anschluss des Differenzdrucksensors 84 ist über den Schlauchaschluss 81 pneumatisch mit der Umgebung verbunden. Der andere Anschluss des Differenzdrucksensors 84 über die Öffnung 99 mit dem Inneren des Strömungsrohrs 72 verbunden. Der Differenzdrucksensor 84 misst also die Druckdifferenz zwischen dem Inneren des Strömungsrohrs 72 und der Umgebung.

Im Unterschied hierzu ist in Fig. 10 ein Schlauch 82 auf den Schlauchanschluss 81 aufgesteckt sowie eine Kapillaröffnung 109 als Bypass zum Differenzdrucksensor 84 vorgesehen. Man kann also über den Schlauch 82 zusätzliche Gase, insbesondere Sauerstoff, dem Strömungsrohr 72 zuführen oder Gasproben aus dem Strömungsrohr 72 ziehen. Über den Differenzdruck an der Kapillaröffnung 109 kann der Gasfluss vom Schlauch 82 in das Strömungsrohr 72 und umgekehrt bestimmt werden.

Die in den Figuren 9 und 10 dargestellten Strömungsrohre 72 können mit ganz ähnlichen Spritzgusswerkzeugen hergestellt werden, die sich lediglich durch einen unterschiedlichen Stempel unterscheiden. Der Stempel zur Herstellung des in Fig. 9 dargestellten Strömungsrohrs 72 ist etwas kürzer, weil ja auch der Hohlraum im Schlauchanschluss 81 etwas kürzer ist. Umgekehrt ist der Stempel zur Herstellung des in Fig. 10 dargestellten Strömungsrohrs 72 ist etwas länger, um auch die Kapillaröffnung 109 herzustellen.

Falls der Differenzdrucksensor 84 in Fig. 9 ein thermisches Sensorelement aufweist, also streng genommen ein Flusssensor ist, kann auch in der in Fig. 9 dargestellten Ausführungsform ein Schlauch 82 auf den Schlauchanschluss 81 aufgesteckt werden, so dass entweder Gase dem Inneren des Strömungsrohrs 72 zugeführt werden können oder Gasproben aus dem Inneren des Strömungsrohrs 72 gezogen werden können.

Obwohl bisher die Erfindung im Zusammenhang mit Gas beschrieben wurde, ist es Fachleuten klar, dass die Flussmessung auch mit Flüssigkeiten funktioniert und dass deshalb der Ausdruck Gas durch den Oberbegriff Fluid ersetzt werden kann.

Die Erfindung wurde zuvor anhand von bevorzugten Ausführungsformen näher erläutert. Für einen Fachmann ist jedoch offensichtlich, dass verschiedene Abwandlungen und Modifikationen gemacht werden können, ohne vom Geist der Erfindung abzuweichen. Deshalb wird der Schutzbereich durch die nachfolgenden Ansprüche und ihre Äquivalente festgelegt.

### Bezugszeichenliste

- 1: Sensorblock
- 2: Strömungsrohr
- 3: Beatmungsschlauchanschluss
- 4: Differenzdrucksensor
- 5: Abdeckung
- 6: Pitotrohr
- 7: Leiterbahn
- 8: Öffnung
- 9: Flussrichtung
- 11, 12: Klammerteil
- 13, 14: Vorsprung
- 15: Nut
- 21: MID-Komponente
- 22: Strömungsrohr
- 24: Differenzdrucksensor
- 25: Abdeckung
- 26: doppeltes Pitotrohr
- 27: Dichtring
- 28: Sterilfilter
- 29: Öffnung
- 32: elektrische Leitung
- 33: Leiter
- 34: Lötstelle
- 36: Öffnung
- 37: Dichtring
- 38: Sterilfilter
- 39: Öffnung
- 41: Schlauchanschluss
- 42: Schlauch
- 44: Differenzdrucksensor
- 46: Öffnung
- 47: Dichtring
- 48: Sterilfilter
- 49: Kapillaröffnung
- 51: elektronische Bauteile für Vorverstärker
- 52: elektronisches Bauteil
- 56, 66: Kanal
- 59, 69: Öffnung
- 72: Strömungsrohr
- 73: elektrischer Anschluss
- 74: Lötstelle
- 76: doppeltes Pitotrohr
- 77: Leiterbahn
- 79: Öffnung
- 81: Schlauchanschluss
- 82: Schlauch
- 84: Differenzdrucksensor
- 86: Blende
- 89, 99: Öffnung
- 101: Sensorblock
- 102: Strömungsrohr
- 109: Kapillaröffnung
- 111, 112: Klammerteil
- 115, 116: Rippe
- 124: Dichtring
- 141: Schlauchanschluss
- 142: Schlauch
- 149: Öffnung

## Patentansprüche

1. Sensorblock (1) zum Messen von Fluidfluss oder Druck in einem Rohr (2; 22; 102) mit:
einem Sensor (4, 24); und
einem Gehäuse,
**dadurch gekennzeichnet, dass**
das Gehäuse einen elastischen Klammerteil (11, 12) aufweist, der so geformt ist, dass er auf das Rohr (2; 22; 102) in radialer Richtung aufgesteckt werden kann und aufgesteckt einen Teil des Rohres (2; 22; 102) umfasst.

2. Sensorblock gemäß Anspruch 1, **dadurch gekennzeichnet, dass** der Klammerteil (11, 12) zwei längliche Vorsprünge (13, 14) aufweist, die sich parallel zur Achse des Rohres (2; 22; 102) erstrecken, wenn der Sensorblock (1) auf das Rohr (2; 22; 102) aufgesteckt ist.

3. Sensorblock gemäß einem der obigen Ansprüche, **dadurch gekennzeichnet, dass** der Sensor ein Differenzdrucksensor (24) ist und im Sensorblock (1) auf der dem Rohr (2; 22; 102) zugewandten Seite zwei Öffnungen (29, 39) aufweist, wobei jede Öffnung mit einem pneumatischen Anschluss eines Differenzdrucksensors (24) pneumatisch verbunden ist.

4. Sensorblock gemäß einem der Ansprüche 1 oder 2, **dadurch gekennzeichnet, dass** das Gehäuse ein doppeltes Pitotrohr (6) aufweist, das zwei Öffnungen (36, 46) in entgegengesetzte Richtungen und zwei Kanäle (56, 66) aufweist, wobei der Sensor ein Differenzdrucksensor (4) ist, wobei jeder Kanal (56, 66) eine Öffnung des doppelten Pitotrohrs mit dem entsprechenden Anschluss des Differenzdrucksensors (4) pneumatisch verbindet, wobei das doppelte Pitotrohr (6) in das Rohr (2; 102) hineinragt, wenn der Sensorblock (1) auf das Rohr (2; 102) aufgesteckt ist.

5. Rohr (72) das mittels MID-Technologie hergestellt ist mit:
Leiterbahnen (77) und
einem Sensor (24, 84), der fest mit dem Rohr (72) verbunden ist.

6. Rohr (72) gemäß Anspruch 5, **dadurch gekennzeichnet, dass** das Rohr (72) ein doppeltes Pitotrohr (76) aufweist, wobei das doppelte Pitotrohr (76) eine erste (46) und eine zweite Öffnung (36) und zwei Kanäle (56, 66) aufweist, wobei die erste Öffnung (46) in Flussrichtung (9) im Rohr (72) und die zweite Öffnung (36) entgegen der Flussrichtung (9) im Rohr (72) gerichtet ist, wobei der Sensor ein Differenzdrucksensor (24) ist, wobei jeder Kanal (56, 66) eine Öffnung (29, 39) des doppelten Pitotrohrs (76) mit dem entsprechenden Anschluss des Differenzdrucksensors (24) pneumatisch verbindet, wobei das doppelte Pitotrohr (76) in das Rohr (72) hineinragt.

7. Gegenstand gemäß einem der Ansprüche 1 oder 5, **gekennzeichnet durch** einen Anschluss (41; 81) für einen Schlauch (42; 82), wobei der Sensor ein Drucksensor (24; 84) ist und pneumatisch mit dem Anschluss (41; 81) verbunden ist, so dass der Drucksensor (24; 84) den Druck im Anschluss (41; 81) misst.

8. Gegenstand gemäß Anspruch 7, **dadurch gekennzeichnet, dass** der Drucksensor ein Differenzdrucksensors (84) mit einem ersten und einem zweiten Anschluss ist, dessen erster Anschluss pneumatisch mit dem Anschluss (81) des Gegenstands verbunden ist und dessen zweiter Anschluss pneumatisch mit dem Inneren des Rohres (72) verbunden ist.

9. Gegenstand gemäß Anspruch 7, **dadurch gekennzeichnet, dass** der Drucksensor ein Differenzdrucksensors (24) mit einem ersten und einem zweiten Anschluss ist, dessen erster Anschluss pneumatisch mit dem Anschluss (41) des Gegenstands verbunden ist und dessen zweiter Anschluss pneumatisch mit der Umgebung des Rohres (22) verbunden ist.

10. Gegenstand gemäß einem der Ansprüche 7 bis 9, **dadurch gekennzeichnet, dass** der Anschluss (41; 81) über eine Kapillaröffnung (49; 109) mit dem Inneren des Rohres (22; 72) verbunden ist.

11. Gegenstand gemäß einem der Ansprüche 1 bis 3 oder 5, **dadurch gekennzeichnet, dass** das Rohr (72) eine Blende (86) aus porösem Material aufweist, wobei das Rohr (72) eine erste Öffnung (79) in Flussrichtung (9) vor der Blende (86) und eine zweite Öffnung (89) in Flussrichtung (9) nach der Blende (86) aufweist, wobei der Sensor ein Differenzdrucksensor (24) mit einem ersten und einem zweiten Anschluss ist, wobei die beiden Anschlüsse des Differenzdrucksensors (24) mit je einer Öffnung (79; 89) des Rohres (72) pneumatisch verbindbar bzw. verbunden sind.

12. Rohr (2; 22) mit:
einer Öffnung (36, 46, 49, 59, 69) in der Rohrwand,
einem Sterilfilter (28, 38, 48), das die Öffnung (36, 46, 49, 59, 69) für größere Partikel, nicht aber für Moleküle, verschließt,
**gekennzeichnet durch**
zwei längliche Nuten (15), die sich parallel zur Achse des Rohres (2; 22) erstrecken, zur lösbaren Befestigung eines Sensorblocks (1).

13. Rohr (22) mit:
einer ersten Öffnung in der Rohrwand,
einem Sterilfilter (38), das die Öffnung für größere Partikel, nicht aber für Moleküle, verschließt,
**dadurch gekennzeichnet, dass**
das Rohr (22) ein doppeltes Pitotrohr (26) aufweist, wobei das Pitotrohr (26) eine erste und eine zweite Öffnung (36, 46), einen ersten Kanal (56) und einen zweiten Kanal (66) aufweist, wobei der erste Kanal (56) die erste Öffnung (36) des Pitotrohres (6) mit der ersten Öffnung in der Rohrwand und der zweite Kanal (66) die zweite Öffnung (46) des Pitotrohres (6) mit einer zweiten Öffnung in der Rohrwand pneumatisch verbindet, wobei die zweite Öffnung in der Rohrwand durch ein zweites Sterilfilter (28) für größere Partikel, nicht aber für Moleküle, verschlossen ist.

14. Rohr (72) mit:
einer ersten Öffnung (79) in der Rohrwand,
einem Sterilfilter (38), das die Öffnung (79) für größere Partikel, nicht aber für Moleküle, verschließt,
**dadurch gekennzeichnet, dass**
das Rohr (72) eine Blende (86) aus porösem Material aufweist, wobei die erste Öffnung (79) in der Rohrwand in Flussrichtung vor der Blende (86) und eine zweite Öffnung (89) im Rohr in Flussrichtung nach der Blende (86) aufweist und die zweite Öffnung (89) in der Rohrwand durch ein zweites Sterilfilter (28) für größere Partikel, nicht aber für Moleküle, verschlossen ist.

15. Herstellungsverfahren mit den Schritten:
Herstellen eines Sensorblocks (1) gemäß einem der Ansprüche 1 bis 4 oder eines Gegenstands gemäß den Ansprüchen 7 bis 9, soweit diese sich auf einen der Ansprüche 1 bis 4 rückbeziehen, mit einem Maskensatz, einem Werkzeugsatz, einem Spritzgusswerkzeug und/oder einem Steuerprogrammteil;
Herstellen eines Rohres gemäß den Ansprüchen 5 und 6 oder einem Gegenstand gemäß den Ansprüchen 7 bis 9, soweit diese sich auf einen der Ansprüche 5 und 6 rückbeziehen, mit dem Maskensatz, dem Spritzgusswerkzeug und/oder dem Steuerprogrammteil.
